# EUROPÄISCHE PATENTSCHRIFT

(11) **EP 0 851 231 B1**
(45) Veröffentlichungstag und Bekanntmachung des Hinweises auf die Patenterteilung: **30.07.2003**
(21) Anmeldenummer: 97122072.8
(22) Anmeldetag: 15.12.1997
(51) Int. Cl.: G01N 33/96, G01N 33/49

(54) **Verwendung von tiefgefrorenem Blut für biologische Untersuchungsverfahren**
Use of frozen blood in a biological test method
Utilisation du sang congelé dans une méthode biologique

(30) Priorität: 23.12.1996 DE 19654266
(43) Veröffentlichungstag der Anmeldung: 01.07.1998
(73) Patentinhaber: Wendel, Albrecht, Prof. Dr., 72070 Tübingen (DE); Hartung, Thomas, Dr. rer. nat. Dr. med., 78464 Konstanz (DE)
(72) Erfinder: Wendel, Albrecht, Prof. Dr., 72070 Tübingen (DE); Hartung, Thomas, Dr. rer. nat. Dr. med., 78462 Konstanz (DE)
(74) Vertreter: TER MEER STEINMEISTER & PARTNER GbR

(56) Entgegenhaltungen:
- WO-A-96/17514
- FR-A- 2 475 737
- US-A- 4 219 440
- US-A- 4 701 417
- US-A- 4 731 330

## Beschreibung

Die Erfindung liegt auf dem Gebiet der Verwendung biologischer Systeme für Untersuchungen. Biologische Systeme werden seit langem in erheblichem Umfang für Untersuchungen verwendet, wobei die Prüfmaterialien. d.h. die zu prüfenden Gegenstände, Stoffe oder Mittel mit dem biologischen System in Kontakt gebracht werden und dann die Antwort des Systems, wozu auch eine Nicht-Antwort gehört, qualitativ und/oder quantitativ erfaßt und gewertet wird. Ganz üblich sind derartige Verfahren in der Dermatologie, wo zur Durchführung eines solchen biologischen Untersuchungsverfahrens als biologisches System die Haut von Probanden oder Hautpräparate verwendet werden und im Kontakt mit einem Prüfmaterial die Antwort der Haut bzw. des Hautpräparates bestimmt wird. Bebrütete Hühnereier werden zur Prüfung auf Schleimhautirritationen, Schlachthofmaterialien aller Art als biologisches System für unterschiedlichste Prüfungen verwendet.

Die vorliegende Erfindung betrifft den speziellen Bereich der Verwendung von Blut als biologischem Untersuchungssystem. Beispielsweise ist es bekannt, daß viele Materialien bei Kontakt mit menschlichem Gewebe, Körperflüssigkeiten und Zellen das Immunsystem aktivieren oder in schädlicher Form überaktivieren können, teils weil sie an sich immunstimulatorisch sind, teils weil sie immunstimulatorische Anteile wie fiebererzeugende Pyrogene enthalten. Zu den Materialien mit einem solchen Gefahrenpotential gehören insbesondere Medikamente, vor allem inhalierbare, injizierbare und infundierbare Produkte, einschließlich Blutersatz- und Blutaustausch-Materialien, Kunststoffe unterschiedlicher Aggregatzustände. Formen und Bestimmungen, einschließlich Dispersionen und Werkstoffen wie Membranen und Prothesen, sowie Naturstoffisolierungen, einschließlich aus Pflanzen, Tier und Mensch oder deren Zell- und Gewebekulturen, isolierten Produkten wie Impfstoffen, Medikamenten und Gentherapeutika. Materialien, die im Kontakt mit menschlichem Gewebe, Zellen oder Körperflüssigkeiten eine Freisetzung von Faktoren aus den Leukozyten wie Zytokinen des Organismus bewirken, werden als Immunaktivatoren bezeichnet, im engeren Sinne als Pyrogene, wenn die freigesetzten Faktoren eine Fieberreaktion im Organismus vermitteln können. Die Produktsicherhelt z.B. im Pharmabereich ist ein dringendes Bedürfnis und macht daher, um auch ausnahmsweise kontaminierte Chargen zu erkennen, ständige Einzelprüfungen an Versuchstieren erforderlich. Statt des Pyrogentests am Kaninchen wie er in den verschiedenen Pharmakopoen vorgeschrieben wird oder des Limulus-Amöbozyten-Lysat-Tests (LAL), der jedoch nur einen Teil der Pyrogene/Immunaktivatoren, nämlich die Endotoxine Gram-negativer Bakterien detektiert, haben die gleichen Erfinder deshalb bereits früher ein Verfahren beschrieben (Europäisches Patent 0 741 294 A2, Hartung und Wendel, ALTEX 12, S .70-75, 1995), bei dem einfach, kostengünstig und mit großer Bandbreite Blut oder Blutaufbereitungen als biologisches System mit dem zu prüfenden Material in Kontakt gebracht und die Freisetzung von Zytokinen z.B. Leukozytenfaktoren wie Interleukin-1 (IL-1β), die im Organismus die Fieberbildung vermitteln (endogene Pyrogene) gemessen wird. Nicht selten wird hierbei aber auch ganz allgemein in Fällen, wo Vollblut als biologisches System für die Untersuchung benötigt wird,die Blut-Antwort baldigst benötigt, so daß die Untersuchung mit dem Prüfmaterial ohne Verzögerung, jedenfalls innerhalb weniger Stunden eingeleitet werden muß. Für ein Austesten der Reaktionsfähigkeit des dabei verwendeten frischen Vollbluts verbleibt damit keine Zeit, so daß eine Verfälschung mit gefährlichen Konsequenzen bzw. die Unbrauchbarkeit der Ergebnisse als Folge eines Bluts eines menschlichen Blutspenders mit abnormen Reaktionen z.B. aufgrund genetischer Variationen. Erkrankungen oder Lebensgewohnheiten, nicht mit der notwendigen Sicherheit ausgeschlossen werden kann. Wie bei dem beispielhaft vorerwähnten Fall der Prüfung pharmazeutischer Produktionschargen ist, bei der Gefahr unsachgemäßen Transports oder Lagerung, oft eine Prüfung vor dem Einsatz des Materials erforderlich, wobei bei der Verwendung von Blut als biologischem System aus vorgenannten Gründen ein Vergleich mit früheren Testergebnissen nicht mit ausreichender Verläßlichkeit möglich ist.

Eine der Aufgaben der Erfindung besteht demgemäß darin, bei der Verwendung von Blut als biologischem System für Untersuchungen einen reproduktionssicheren, störungsfreien Ablauf zu sichern, die Möglichkeit von Verfälschungen aufgrund abnormer Reaktionen auszuschließen und einen Weg aufzuzeigen, der die Vergleichbarkeit von Untersuchungsergebnissen mit dem gleichen Prüfmaterial zuläßt. Es soll die wiederholte Prüfung standardisiert an verschiedenen Orten und zu verschiedenen Zeiten ermöglicht werden. Weitere Aufgaben ergeben sich aus den weiterhin geltend gemachten Vorteilen.

Die Lösung der gestellten Aufgabe gelingt durch die Verwendung von tiefgefrorenem. Kryopräservierungsmittel enthaltendem menschlichem oder tierischem Blut oder Blutzubereitungen für ein biologisches Untersuchungsverfahren zur Bestimmung der Blut-Antwort nach Kontakt des aufgetauten Bluts oder aufgetauten Blutzubereitung mit Prüfmaterial und Inkubation.

Die Verwendung tiefgefrorenen Bluts bzw. einer ein solches Blut enthaltenden Zubereitung ermöglicht die jederzeitige Verfügungsbereitschaft eines zuvor ausgetesteten und damit von abnormen Reaktionen freien Bluts als biologischem System, zumindest aber eine Standardisierbarkeit und damit die Verwendung eines standardisierten Reagenzes. Nachdem von einer Blutcharge regelmäßig eine Vielzahl voll identischer, tiefgefrorener Einheiten herstellbar ist, kann damit der Forderung nach Reproduzierbarkeit der Untersuchungen zu verschiedenen Zeiten an verschiedenen Orten Genüge getan werden, abgesehen davon, daß darüberhinaus eine Charakterisierung einer solchen Charge mit untersuchungsrelevanten Daten auch Wege für Vergleiche mit Untersuchungsergebnissen bei Verwendung einer anderen, ebenfalls ausreichend charakterisierten Blutcharge eröffnet. Es ist zwar aus der US-A-4 731 330 ein Eichpulver zur Eichung von Vorrichtungen bekannt. Abgesehen von der Verschiedenheit in aufgabe und Lösung ist dieses denaturierte Eichmaterial für die erfindungsgemäße Verwendung untauglich.

Für das tiefgefrorene Blut hat sich tierisches oder menschliches Vollblut ohne Trennung von Einzelkomponenten, besonders frisch gewonnenes Blut gesunder menschlicher Spender bewährt. Die einzelnen Bestandteile einschließlich Leukozyten liegen so in ihrer natürlichen Umgebung vor, einschließlich aller Serumkomponenten, die auf die Wirkung des Prüfmaterials möglicherweise Einfluß haben. Die Verwendung tiefgefrorenen Vollbluts ist deshalb weit bevorzugt, wenn auch die Verwendung von aus Blut isolierten Leukozyten nicht ausgeschlossen ist. Die meisten Isolierungsmethoden sind jedoch für eine Routinetestung sehr aufwendig, so daß die Verwendung von Vollblut einfacher und für die gewünschte Aussage sicherer und wegen der interaktiven Einflußmöglichkeit der weiteren Blutbestandteile aussagekräftiger ist.

In jedem Fall ist zur Durchführung des biologischen Untersuchungsverfahrens das tiefgefrorene Produkt aufzutauen. Das Prüfmaterial wird mit dem Blut bzw. dessen Zubereitungen solange und in solcher Weise in Kontakt gebracht, wie dies für die Erzielung einer ausreichenden Blut-Antwort erforderlich ist, Kontaktzeiten von einigen Sekunden oder Minuten können ausreichend sein, ohne daß mehrstündige Kontaktzeiten ausgeschlossen wären. Jede der zur Erreichung des vorgenannten Zwecks geeignete Kontaktform kann angewendet werden, z.B. bei Gegenständen das Eintauchen, Durchspülen, Ablaufenlassen, bei Lösungen, Dispersionen, Kulturen und Feststoffen das Vermischen mit oder Zusetzen von oder zu dem Blut bzw. den Blutzubereitungen und dergleichen. Zur Ermittlung der Blut-Antwort, regelmäßig nach auch kurzzeitiger Inkubation des Bluts bzw. der Blutzubereitung nach dem Kontakt mit dem Prüfmaterial, können die üblichen biologischen, physikalischen, chemischen und/oder physikalisch-chemischen Bestimmungs- und Meßmethoden verwendet werden. Hierzu gehören biologische Assays- und RIA-Verfahren, bevorzugt spektrometrische Verfahren, alle Verfahren, die der qualitativen und quantitativen Stoffmessung freigesetzter oder gebildeter Stoffe dienen wie ELISA, weiter Verfahren der Turbodimetric, Chemoluminiszenz usw.

Die Zubereitungen mit tiefgefrorenem Blut oder auch Leukozyten können Verdünnungsmittel, gerinnungsverzögernde Bestandteile, Kryopräservierungsmittel und andere an sich bekannte sofern das Untersuchungsergebnis nicht beeinflussende Zusätze enthalten. Als Verdünnungsmittel haben sich isotonische Lösungen wie isotonische Kochsalzlösung, Ringer'sche Lösung und Zellkulturmedien wie RMPI 1640 bewährt. Es ist von Vorteil, die Verdünnungsmittel erst zur Testdurchführung während oder nach dem Auftauen z.B. bei 37°C zuzusetzen. Die Verdünnungsmittel können beispielsweise in Mengen von 5 - 95 Vol.-% in der Gesamtzubereitung enthalten sein. Häufig wird eine Verdünnung 1 zu 10 Vol.-Teilen Verdünnung benutzt.

Zur Herstellung des tiefgefrorenen Bluts-Reagens wird das Blut oder die Blutzubereitung mit Kryopräservierungsmitteln versetzt, gegebenenfalls geeignet portioniert und dann tiefgefroren. Als solche Kryopräservierungslösungen eignen sich organische Lösungen, anorganische, auch salzhaltige Lösungen oder deren Mischungen in variablen Anteilen. So hat sich zum Beispiel die Verwendung von 10 % Dimethylsulfoxid allein oder von Glycol bzw. Glycerin allein oder in Mischungen untereinander oder mit Dimethylsulfoxid als besonders geeignet erwiesen. Vorteilhaft ist ein gesteuertes langsames Einfrieren zum Beispiel mit -1°C pro Minute. Bewährt hat sich eine Lagertemperatur des gefrorenen Blutes bei -70°C im Tiefkühlschrank, oder auch der Einsatz kondensierter oder fester Gase wie flüssiger Stickstoff oder Trockeneis. Letzteres eignet sich besonders zum Transport.

Gerinnungsverzögernde Bestandteile können sowohl in der tiefgefrorenen Zubereitung enthalten sein als auch während und nach dem Auftauen und während der Durchführung des Untersuchungsverfahrens z.B. während einer etwaigen Inkubation zugesetzt werden.

Beispiele geeigneter Gerinnungshemmer sind Natriumzitrat z.B. einer Endkonzentration von 0,38 Gew.-% oder Heparin wie Natriumheparin, Heparinfraktionen und dergleichen.

Die erfindungsgemäße Verwendung von tiefgefrorenes Blut enthaltenden Zubereitungen einschließlich tiefgefrorenem Blut oder tiefgefrorenen Leukozyten wird mit besonderem Vorteil zur Untersuchung von Materialien, wie den eingangs schon erörterten Gegenständen, Stoffen und Mitteln auf immunstimulatorische oder immunmodulatorische Wirkungen bzw. zur qualitativen und/oder quantitativen Bestimmung immunrelevanter Wirkungen verwendet, unter Ermittlung, Bestimmung bzw. Auswertung der Blut-Antwort bei Kontakt dieser Prüfmaterialien mit dem aufgetauten, tiefgefrorenen Blut bzw. Blutzubereitung.

Über immunstimulatorische z.B. pyrogene Wirkungen hinaus können Materialien die nachfolgende Aktivierung des Immunsystems modulieren, was bei Immuntherapeutika erwünscht aber auch häufig eine unerwünschte toxische Wirkung ist. Eine solche Wirkung soll bei der immunpharmakologischen Wirkstoffindung gezielt gesucht bei vielen anderen Stoffen jedoch ausgeschlossen werden. Von therapeutischem Interesse sind einerseits Immunstimulanten zur Therapie absoluter oder relativer Immunschwäche und andererseits Immunsuppressiva und Entzündungshemmer, einschließlich der Antiphlogistika, Antirheumatika, Antiallergika. Immuntoxikologische Wirkungen von Materialien z.B. von Substanzen kommen erst in den letzten Jahren zunehmend in das öffentliche Bewußtsein. Für die immunpharmakologische wie immuntoxikologische Stoffprüfung gibt es bisher keine standardisierten Testsysteme. Stimuliertes Vollblut wird jedoch zunehmend wissenschaftlich zur Charakterisierung der pharmakologischen Eigenschaften von Wirkstoffen eingesetzt [Hartung et al. Blood 85 (1995) 2482-2489].

Gerade auch bei der Untersuchung auf immunrelevante Wirkungen wie bei der Bestimmung von Pyrogenen, Immunmodulatoren und Immuntoxinen ist das zentrale Problem der Anwendungen von Blut und seiner Faktorfreisetzung die Standardisierung des verwendeten Bluts.

Auch hier können die vorgenannten Blutzubereitungen wie Vollblut, gegebenenfalls verdünnt, zum Beispiel mit Zellkulturmedien, Puffern oder klinischer Kochsalzlösung, verwendet werden. Hier muß besonders häufig die Durchführung der Untersuchung innerhalb weniger Stunden beginnen, so daß eine vorausgehende Prüfung auf Eignung des verwendeten Blutmaterials deshalb im allgemeinen nicht möglich ist. In besonderem Maße stellt sich die vorgenannten Aufgabe demgemäß bei der Untersuchung von Materialien auf immunrelevante Wirkungen wie auf pyrogene, immunmodulatorische oder immuntoxische Wirkungen und soll die vorherige Prüfung des Bluts sowie die wiederholte Prüfung eines Materials an verschiedenen Orten und zu verschiedenen Zeiten mit demselben Test-Blut ermöglicht werden. Dies wird durch die erfindungsgemäße Verwendung zuverläßig bewirkt.

Der besondere Vorteil der Erfindung besteht bei der Ermittlung immunrelevanter Wirkungen und Daten in der Verwendung eines biologischen Systemes, das relevante Aussagen für die Exposition des Menschen liefert. Als Meßparameter dienen Zytokine wie die endogenen Pyrogene Interleukin-1β. Interleukin-6 und Tumor-Nekrose-Faktor. Eicosanoide wie das endogene Pyrogen Prostaglandin E₂, oder andere von Leukozyten freigesetzte Stoffe wie Degranulationsprodukte, lösliche Rezeptoren, Proteine oder niedermolekulare Substanzen (vgl. auch EP 0 741 294 A2, woraufBezug genommen wird). Bemerkenswert ist, daß auch in vorliegendem Verwendungsverfahren nicht nur die Gerinnungshemmer Citrat und Heparin keinen das Ergebnis verfälschenden Einfluß haben, sondern auch die Tieffrierkonservierung einschließlich der genannten Kryopräservierungsmittel zu einem Blut führt, das nach dem Auftauen keinerlei nachteilige Auswirkungen auf das Untersuchungsergebnis zeigt.

Die Erfindung weist eine ganze Reihe von weiteren Vorteilen auf. Nach dem Auftauen des tiefgefrorenen Bluts werden wieder körpereigene Reaktionen von nativen Immunzellen unter natürlichen Bedingungen und Mischungsverhältnissen erfaßt.

Es kann dank der Tieffrier-Konservierung wiederholt dasselbe Blut zu verschiedenen Zeiten und an verschiedenen Orten verwendet werden. Durch entsprechende Vortestungen können abnorme Blutreaktionen erkannt und das entsprechende Material ausgesondert werden. Es können vorab unter standardisierten Bedingungen Normwerte für die jeweilige Blutcharge ermittelt werden. Es ermöglicht die Testdurchführung auch ohne unmittelbare Möglichkeiten zur Blutabnahme. Es können nicht nur z.B. direkte Immunaktivatoren sondern auch Immunmodulatoren/Immuntoxine an ihrer Modulation der Wirkung eines Standardstimulus wie Endotoxin (Lipopolysaccharid Gram-negativer Bakterien) ermittelt werden. Die Erfindung eignet sich auch zur Prüfung von pharmakologischen Therapien oder Expositionen ex vivo, indem Blut entsprechender Patienten, Probanden oder Versuchstiere nach Verabreichung gewonnen und entsprechend tiefgefroren z.B. kryopräserviert wird und erst später z.B. die Reaktionsfähigkeit gegen einen Standardstimulus getestet wird.

Gegenstand der Erfindung ist auch die Verwendung von tiefgefrorenen Kryopräservierungsmittel aufweisenden Blut oder Blutzubereitung enthaltenden standardisierten Einheitsdosen für biologische Untersuchungsverfahren zur Bestimmung der Blut-Antwort nach Kontakt des aufgetauten Bluts oder Blutzubereitung mit Prüfmaterial und Inkubation.

Als Blut wird bevorzugt frisches Vollblut von gesunden tierischen oder menschlichen Spendern verwendet, das gegebenenfalls nach Zusatz von Kryopräservierungsmitteln, Verdünnungs- und/oder gerinnungshemmenden Mitteln, wie sie in beispielhafter Weise zuvor beschrieben sind, portioniert und tiefgefroren wird. Die Einheitsdosis enthält meist zwischen 50 bis 500 Mikroliter, bevorzugt 100 Mikroliter Vollblut, ohne darauf beschränkt zu sein. Zur Durchführung des Untersuchungsverfahrens wird das tiefgefrorene Blut einer Einheitsdosis aufgetaut, z.B. bis auf ein gewünschtes Volumen, beispielsweise 1 Milliliter, mit Verdünnungsmitteln z.B. vorgenannter Art aufgefüllt, mit dem Prüfmaterial in Kontakt gebracht und die Blut-Antwort in üblicher Weise bestimmt und ausgewertet. Das üblicherweise von einem gesunden menschlichen Spender in einer Sitzung abnehmbare Vollblutvolumen erlaubt bei einer Einzeldosis von z.B. 100 Mikroliter Blut die Herstellung von mehreren tausend solcher Einheitsdosen. Die Einheitsdosen können zu Kollektiven von beispielsweise 5, 10 oder mehr Einheitsdosen zusammengefaßt und, gegebenenfalls versehen mit für das Untersuchungsverfahren verwertbaren Daten, unter Aufrechterhaltung der Tiefkühlung in den Verkehr gebracht werden. Die Kollektive können für besondere Verwendungszwecke neben Einheiten mit Einheitsdosen auch Einheiten mit einem mehrfachen der Einheitsdosis enthalten.

Die jeweilige Einheitsdosis kann im übrigen die gleichen Bestandteile ent halten und die gleiche Zusammensetzung aufweisen sowie in gleicher Weise hergestellt sein und verwendet werden wie die zuvor beschriebenen tiefgefrorenes Blut enthaltenden Zubereitungen.

### Beispiel

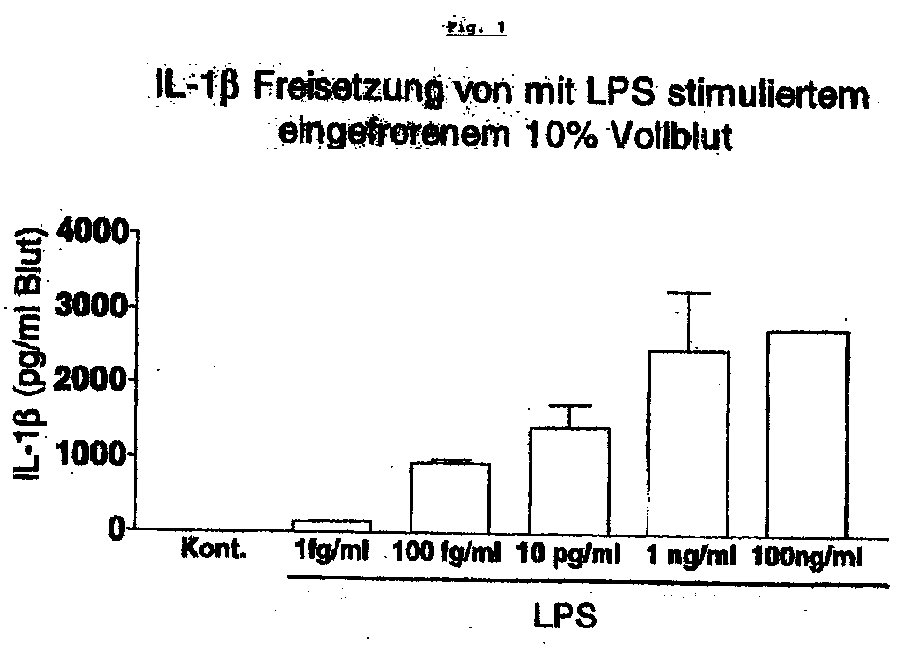

Zitratblut gesunder Spender wurde unmittelbar nach Abnahme mit 10 % Dimethylsulfoxid (Merck, Darmstadt) versetzt, zu jeweils 100 µl in 2-ml Reaktionsgefäße aliquotiert (Eppendorf, Hamburg) und in kommerziellen Einfriersystemen (Mr. Freezy, Nalgene) in einem -70°C-Tiefkühlschrank überführt. Das Auftauen erfolgt in einem Thermoschüttler (Eppendorf, Hamburg) bei 37°C. Unmittelbar nach dem Auftauen werden jeweils 900 µl 37°C-warmes RPMI 1640 (Gibco, Eggenstein) zugesetzt. Anschließend werden die Prüfsubstanzen zum Beispiel Verdünnungen eines Pyrogens, hier Lipopolysaccharid von Salmonella abortus equi (Sigma, Deisenhofen) zugesetzt. Nach einer vierstündigen Inkubation bei 37°C zum Beispiel im Brutschrank (Heraeus, Fellbach) mit 5 % Kohlendioxid, werden die Inkubationen geschüttelt, abzentrifugiert. Im zellfreien Überstand werden ggf. nach Einfrieren endogene Pyrogene, hier IL-1β bestimmt. Abb. 1 zeigt IL-1β-Bildung im kryopräservierten Blut von vier gesunden Spendern in Abhängigkeit der zugesetzten Pyrogenmenge. IL-1β wurde mit einem ELISA (enzyme-linked immunsorbent asay) aus Antikörpern der Firma Pharmingen (Hamburg) gemessen. Der Versuch zeigt die Freisetzung endogener Pyrogene bei Anwesenheit geringster Pyrogenmengen.

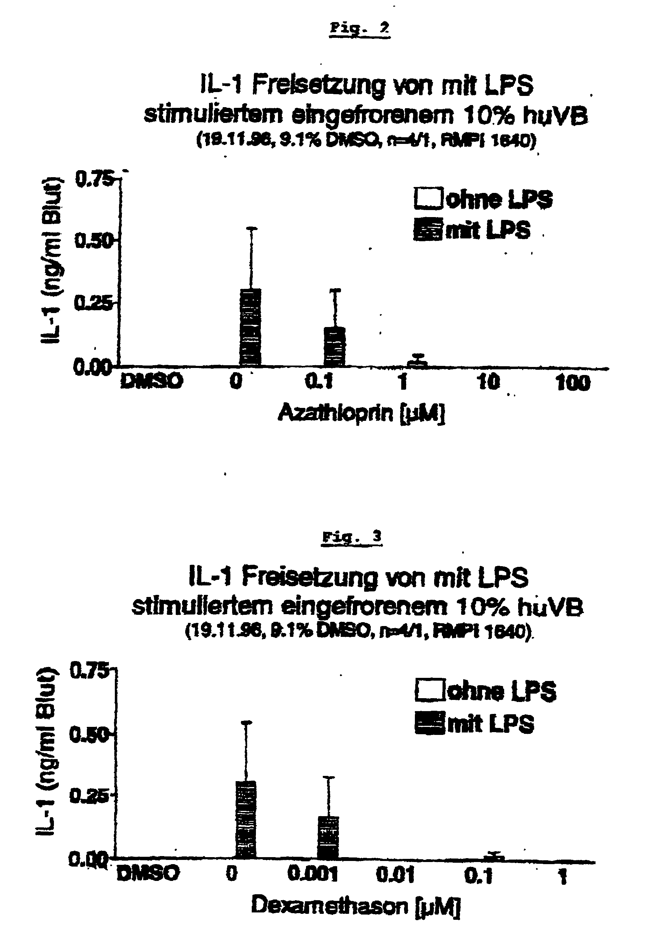

Die Figuren 2 und 3 zeigen die Freisetzung von IL-1 bei mit LPS stimuliertem, eingefrorenem Vollblut in Abhängigkeit von der jeweils verabfolgten Menge an Azathioprin bzw. Dexamethason.

## Patentansprüche

1. Verwendung von tiefgefrorenem, Kryopräservierungsmittel enthaltendem menschlichem und tierischem Blut oder einer solches enthaltenden Blutzubereitungen für ein biologisches Untersuchungsverfahren zur oder mit Bestimmung der Blut-Antwort nach Kontakt des aufgetauten Bluts oder aufgetauten Blutzubereitung mit Prüfmaterial und Inkubation.

2. Verwendung gemäß Patentanspruch 1, wobei als Blut eine tiefgefrorene aus Blut isolierte Leukozyten enthaltende Zubereitung verwendet wird.

3. Verwendung gemäß einem der vorhergehenden Patentansprüche, worin die Zubereitung Gerinnungshemmer enthält.

4. Verwendung gemäß einem der vorhergehenden Patentansprüche zur Ermittlung immunrelevanter Daten.

5. Verwendung von tiefgefrorenem, Kryopräservierungsmittel aufweisendem Blut oder einer solches enthaltenden Blutzubereitung in Form von standardisierten Einheitsdosen für biologische Untersuchungsverfahren zur oder mit Bestimmung der Blut-Antwort nach Kontakt des aufgetauten Bluts oder Blutzubereitung mit Prüfmaterial und Inkubation.

6. Verwendung von standardisierten Einheitsdosen gemäß Patentanspruch 5 enthaltend neben Einheiten jeweils mit standardisierter Einheitsdosis auch Einheiten jeweils mit einem mehrfachen der Einheitsdosis.

7. Verwendung gemäß Patentanspruch 5 oder 6, worin die Zubereitungen Gerinnungshemmer und/oder Verdünnungsmittel enthalten.

8. Verwendung gemäß einem der vorhergehenden Patentansprüche zur Ermittlung immunrelevanter Daten.

## Claims

1. Use of deep-frozen, cryopreservative-containing human and animal blood or of a blood preparations containing such for a biological investigation method for or with determination of the blood response after contact of the thawed blood or thawed blood preparation with test material and incubation.

2. Use according to Claim 1, where a deep-frozen preparation containing leukocytes isolated from blood is used as blood.

3. Use according to either of the preceding claims, in which the preparation contains anticoagulant.

4. Use according to any of the preceding claims for the measurement of immunorelevant data.

5. Use of deep-frozen blood including cryopreservative or of a blood preparation containing such in the form of standardized unit doses for biological investigation methods for or with determination of the blood response after contact of the thawed blood or blood preparation with test material and incubation.

6. Use of standardized unit doses according to Claim 5 containing besides units each with a standardized unit dose also units each with a multiple of the unit dose.

7. Use according to Claim 5 or 6, in which the preparations contain anticoagulants and/or diluents.

8. Use according to any of the preceding claims for the measurement of immunoresevant data.

## Revendications

1. Utilisation de sang humain et animal congelé contenant des agents de préservation cryogénique, ou d'une préparation sanguine contenant un tel sang, pour un procédé d'examen biologique pour ou avec détermination de la réponse du sang après contact du sang décongelé ou de la préparation sanguine décongelée avec un matériau de test, et incubation.

2. Utilisation selon la revendication 1, dans laquelle on utilise en tant que sang une préparation congelée contenant des leucocytes isolés à partir de sang.

3. Utilisation selon l'une des revendications précédentes, dans laquelle la préparation contient des anti-coagulants.

4. Utilisation selon l'une des revendications précédentes pour obtenir des données pertinentes sur le plan immunitaire.

5. Utilisation de sang congelé comportant des agents de préservation cryogénique, ou d'une préparation sanguine contenant un tel sang, sous la forme de doses unitaires standardisées pour des procédés d'examen biologiques pour ou avec détermination de la réponse du sang après contact du sang décongelé ou de la préparation sanguine décongelée avec un matériau de test, et incubation.

6. Utilisation de doses unitaires standardisées selon la revendication 5, incluant à côté d'unités chacune avec une dose unitaire standardisée, également des unités comprenant chacune un multiple de la dose unitaire.

7. Utilisation selon la revendication 5 ou 6, dans laquelle les préparations contiennent des anti-coagulants et/ou des solvants.

8. Utilisation selon l'une des revendications précédentes, pour obtenir des données pertinentes sur le plan immunitaire.
